(19) Europäisches Patentamt
European Patent Office
Office européen des brevets

(11) EP 0 514 338 B1

(12) **EUROPÄISCHE PATENTSCHRIFT**

(45) Veröffentlichungstag und Bekanntmachung des Hinweises auf die Patenterteilung:
**27.03.1996 Patentblatt 1996/13**

(51) Int. Cl.⁶: **C07C 13/72**, C07C 2/86

(21) Anmeldenummer: **92810344.9**

(22) Anmeldetag: **08.05.1992**

# (54) Gemische aus Spiroheptadienverbindungen

Mixtures of sprioheptadiene compounds

Mélanges de composés spiro-heptadiène

(84) Benannte Vertragsstaaten:
**CH DE ES FR GB IT LI NL SE**

(30) Priorität: **17.05.1991 CH 1476/91**
**15.07.1991 CH 2096/91**

(43) Veröffentlichungstag der Anmeldung:
**19.11.1992 Patentblatt 1992/47**

(73) Patentinhaber: **CIBA-GEIGY AG**
**CH-4002 Basel (CH)**

(72) Erfinder:
- **Zahir, Sheik Abdul-Cader, Dr.**
  **CH-4104 Oberwil (CH)**
- **Pasquier, Cécile, Dr.**
  **CH-1623 Semsales (CH)**
- **Scharf, Wolfgang, Dr.**
  **W-7889 Grenzach-Wyhlen (DE)**
- **Dupré, Maurice**
  **CH-1723 Marly (CH)**

(56) Entgegenhaltungen:
**DE-B- 1 643 855** **US-A- 2 726 232**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 514 338 B1

Printed by Rank Xerox (UK) Business Services
2.9.14/3.4

## Beschreibung

Gegenstand vorliegender Erfindung sind Gemische aus neuen Spiro[2,4]hepta-4,6-dien-verbindungen auf Basis aromatischer Bis-(halogenmethyl)-verbindungen, Verfahren zu deren Herstellung und deren Verwendung, insbesondere als Laminierharze.

Dimere und oligomere Cyclopentadiene auf Basis von aromatischen Bis-(chlormethyl)-verbindungen sind bekannt. Beispielsweise werden im US-Patent 2,726,232 und in der DE-Auslegeschrift 16 43 855 unsubstituierte und substituierte, dimere und oligomere Cyclopentadiene auf Basis von aromatischen Bis-(cyclopentadienyl)-xylolen und deren Verwendungen, unter anderem auch als Laminierharze, beschrieben. Diese bekannten Harze weisen nur eine geringe Lagerstabilität auf, so dass die daraus hergestellten Prepregs gleich zu Pressformstoffen verarbeitet werden müssen.

Es wurde nun gefunden, dass man durch Umsetzung der bekannten aromatischen Bis-(cyclopentadienyl)-verbindungen mit 1,2-Dichlor- oder 1,2-Dibromethan neue Spiro-[2,4]hepta-4,6-diene erhält, die vergleichsweise eine bessere Lagerstabilität aufweisen, insbesondere oxidationsstabiler sind, und somit durch bessere Verarbeitungseigenschaften, insbesondere für die Laminat-bzw. Pressformstoffherstellung, gekennzeichnet sind. Die ausgehärteten Spiro[2,4]hepta-4,6-diene weisen vorteilhaft eine Dielektrizitätskonstante von unterhalb 3 auf, so dass sie als Laminierharze für die Herstellung von gedruckten Schaltungen, die in der Hochfrequenztechnik eingesetzt werden, besonders geeignet sind.

Gegenstand vorliegender Erfindung sind somit Gemische enthaltend im wesentlichen Verbindungen der Formel I oder II

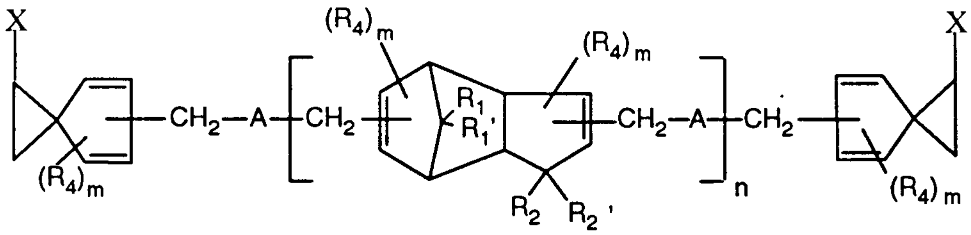

(I) oder

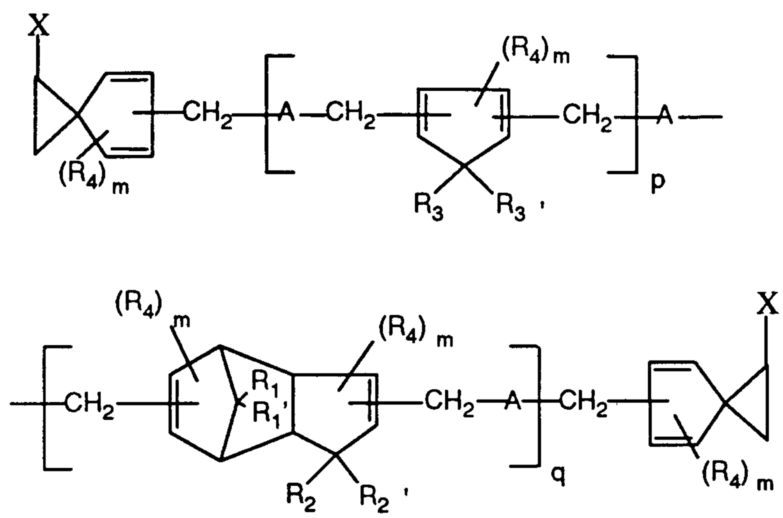

(II),

worin X für ein Wasserstoffatom oder Vinyl steht, A einen unsubstituierten oder durch ein oder mehrere $C_1$-$C_{20}$-Alkyle, Halogenatome oder halogensubstituierte $C_1$-$C_{20}$-Alkyle substituierten zweiwertigen aromatischen Rest bedeutet, $R_1$, $R_1'$, $R_2$, $R_2'$, $R_3$ und $R_3'$ je für ein Wasserstoffatom oder $R_1$ und $R_1'$, $R_2$ und $R_2'$ und $R_3$ und $R_3'$ zusammen je für Ethylen, $R_4$ je für ein $C_1$-$C_6$-Alkyl, Phenylalkyl mit 1-6 C-Atomen im Alkylrest oder Trialkylsilyl mit je 1-6 C-Atomen im Alkylrest, m für Null oder die Zahl 1 oder 2, n für Null oder eine durchschnittliche Zahl von 1 bis 200, p für eine durchschnittliche Zahl von 1 bis 100 und q für Null oder eine durchschnittliche Zahl von 1 bis 100 stehen.

Vorzugsweise steht X in den Formeln I und II für ein Wasserstoffatom.

Insbesondere enthalten die erfindungsgemässen Gemische im wesentlichen Verbindungen der Formel I, worin A, $R_1$, $R_1'$, $R_2$, $R_2'$, $R_4$, m und n die zuvor angegebene Bedeutung haben.

Bei dem zweiwertigen aromatischen Rest A in den erfindungsgemässen Gemischen der Formeln I und II kann es sich um einen einkernigen oder mehrere Benzolkerne umfassenden Rest handeln, worin die Benzolkerne kondensiert vorliegen können oder über eine direkte Bindung oder über Brückenglieder miteinander verbünden sind und worin die aromatischen Ringe gleiche oder verschiedene Substituenten aufweisen können.

Beispielsweise kann A für einen Rest der folgenden Formeln stehen:

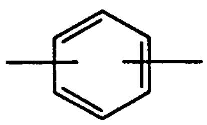 , 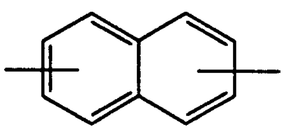 , 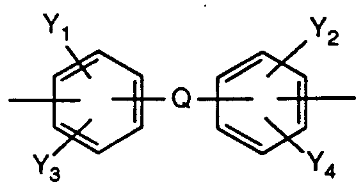 ,

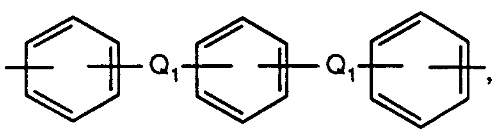 , 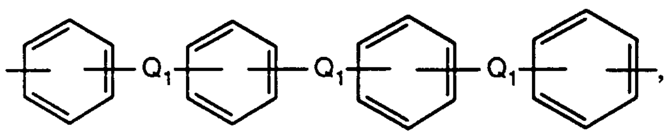 ,

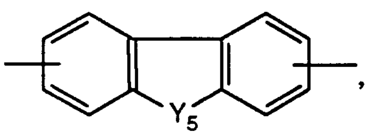 , 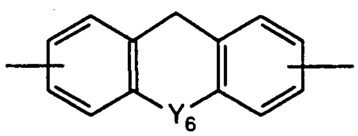 oder 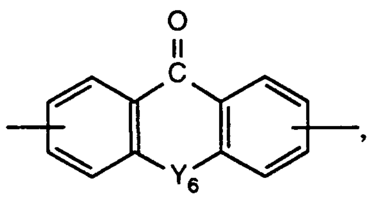 ,

worin Q die direkte Bindung, $-(CH_2)_r-$, $-CH_2CH_2-$, $-CH(CH_3)-$, $-C(CH_3)_2-$, $-C(CF_3)-$, -O-, -S-, $-SO_2-$, oder -CO- ist, wobei r für eine Zahl von 1 bis 12 steht, $Q_1$ die direkte Bindung, $-CH_2-$, $-CH_2-CH_2$, $-CH(CH_3)-$, $-C-(CF_3)-$, -O-, -S-, $-SO_2-$ oder -CO- ist, n 2-12 ist, $Y_1$, $Y_2$, $Y_3$ und $Y_4$ unabhängig voneinander je ein Wasserstoffatom, Halogenatom oder $C_1$-$C_4$-Alkyl bedeuten, $Y_5$ für $-CH_2-$, -O-, -S-, -NH- oder -CO- und $Y_6$ für $-CH_2-$, -O-, -S- oder -NH- stehen.

Die $C_1$-$C_{20}$-Alkyle als Substituenten des aromatischen Restes A und die $C_1$-$C_6$-Alkyle als Reste $R_4$ können linear oder verzweigt sein. Beispiele für solche Reste sind Methyl, Ethyl, n-Propyl, Isopropyl, n-Butyl, tert.-Butyl, n-Pentyl, Isopentyl, Neopentyl, 2,2-Dimethylpentyl, 2-Methylhexyl, 2,2,3-Trimethylbutyl, Isooctyl und Dodecyl.

Als Halogenatome können beispielsweise Fluor, Chlor, Brom oder Jod am aromatischen Ring substituiert sein. Vorzugsweise sind an den aromatischen Ringen Fluor oder Chlor als Halogenatome substituiert.

Als halogensubstituierte $C_1$-$C_{20}$-Alkyle sind beispielsweise Chlormethyl, 2-Bromethyl, 3-Chlor-n-propyl, Chlorisopropyl oder 5-Chlor-n-pentyl und besonders Perfluoralkyle geeignet.

Geeignete Phenylalkyle sind beispielsweise Phenylethyl oder Phenylpropyl und insbesondere Benzyl.

Als Trialkylsilyl sind vorzugsweise Trimethyl- oder Triethylsilyl geeignet.

Vorzugsweise enthalten die erfindungsgemässen Gemische Verbindungen der Formel I oder II, worin A für einen unsubstituierten oder einen $C_1$-$C_4$-alkylsubstituierten Rest der Formel

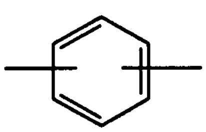 , 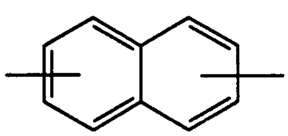 oder 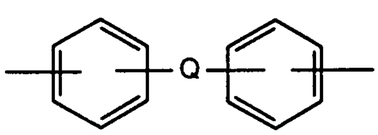

steht, worin Q die direkte Bindung, $-(CH_2)_r-$, $-C(CH_3)_2-$, -O, -CO-, -S- oder $-SO_2-$ bedeutet, wobei r für eine Zahl von 1 bis 12 steht, $R_1$, $R_1'$, $R_2$ und $R_2'$ die zuvor genannte Bedeutung haben, m für Null oder die Zahl 1 oder 2 steht, wobei $R_4$ je ein Methyl oder Ethyl bedeutet, und n für Null oder eine durchschnittliche Zahl von 1 bis 20 steht.

Insbesondere enthalten die erfindungsgemässen Gemische Verbindungen der Formel I oder II, worin A für Phenylen oder Biphenylen steht, $R_1$, $R_1'$, $R_2$ und $R_2'$ die zuvor genannte Bedeutung haben und n für Null oder eine durchschnittliche Zahl von 1 bis 20, vorzugsweise 1 bis 10, steht.

Die erfindungsgemässen Gemische enthaltend Verbindungen der Formel I oder II können beispielsweise hergestellt werden, indem man Verbindungen der Formel III oder IV

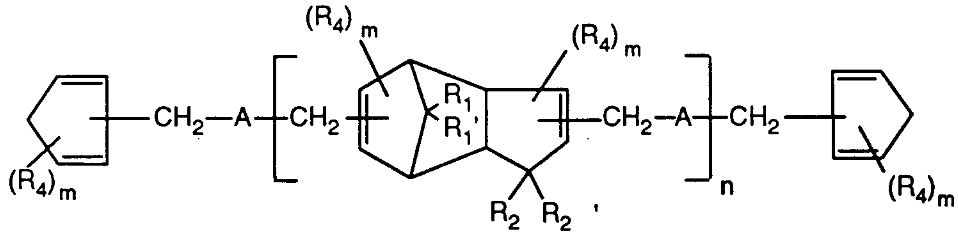

(III) oder

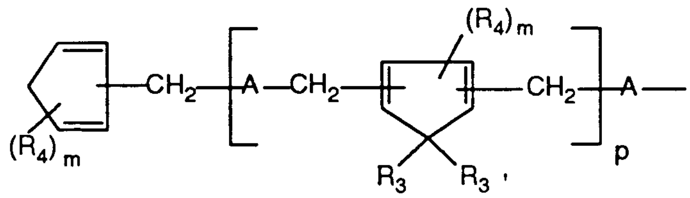

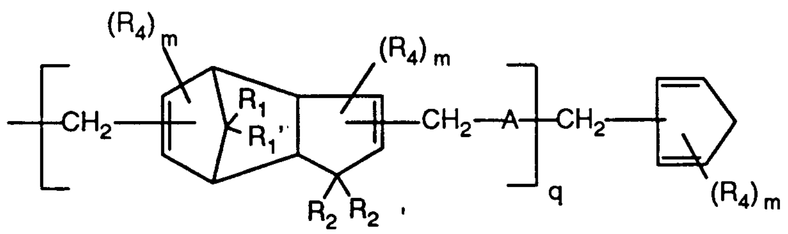

(IV),

worin A, $R_1$, $R_1'$, $R_2$, $R_2'$, $R_3$, $R_3'$, $R_4$, m, n, p und q die zuvor geuannte Bedeutung haben, mit 1,2-Dichlor-, 1,2-Dibromethan, 1,4-Dichlor- oder 1,4-Dibrombuten-(2) in Gegenwart eines Phasentransferkatalysators und von Alkali im Temperaturbereich von 20 bis 50°C umsetzt, wobei man pro 1 Mol der Verbindung der Formel III oder IV mindestens 0,1 Mol 1,2-Dichlor-, 1,2-Dibromethan, 1,4-Dichlor- oder 1,4-Dibrombuten-(2) einsetzt.

Vorzugsweise setzt man bei diesem Verfahren mindestens 2 Mol, im allgemeinen 2 bis 10 Mol, 1,2-Dichlor-, 1,2-Dibromethan, 1,4-Dichlor- oder 1,4-Dibrombuten-(2) pro 1 Mol der Verbindung der Formel III oder IV ein.

Verbindungen der Formel III, worin $R_1$, $R_1'$, $R_2$ und $R_2'$ je für ein Wasserstoffatom stehen, sind bekannt, beispielsweise aus dem US-Patent 2,726,232 oder aus der DE-Auslegeschrift 1 643 855, und können hergestellt werden, indem man 1 Mol einer Verbindung der Formel V

$$X\text{-}CH_2\text{-}A\text{-}CH_2\text{-}X \qquad (V),$$

worin X für ein Halogenatom, vorzugsweise Chlor oder Brom, oder für den Rest $-OSO_2-R$, wobei R ein $C_1$-$C_4$-Alkyl darstellt, steht, mit 2 Mol Cyclopentadien in Gegenwart eines Phasentransferkatalysators und Natronlauge im Temperaturbereich von 25 bis 50°C umsetzt, wobei erst Verbindungen der Formel III, worin n gleich Null ist, erhalten werden, die dann durch Erwärmen auf etwa 50 bis 100°C durch eine Diels-Alder-Reaktion zu Verbindungen der Formel III, worin n eine Zahl von 1 bis 200 ist, polymerisiert werden können.

Die Verbindungen der Formel III, worin $R_1$ und $R_1'$ sowie $R_2$ und $R_2'$ auch zusammen je für ein Ethylen stehen, $R_4$, m und A die zuvor angegebene Bedeutung haben und n für eine durchschnittliche Zahl von 1 bis 200 steht, sind in der Literatur noch nicht beschrieben worden und können durch Diels-Alder Reaktion von Verbindungen der Formel I mit Verbindungen der Formel III, worin n jeweils Null ist, bei erhöhter Temperatur, beispielsweise zwischen 50 und 100°C, erhalten werden. Solche Reaktionsgemische liegen beispielsweise vor, wenn man bei der Herstellung von Verbindungen der Formel I, worin n gleich Null ist, das 1,2-Dichlor-, 1,2-Dibromethan, 1,4-Dichlor- oder 1,4-Dibrombuten-(2) in geringeren als erforderlichen Mengen einsetzt, d.h., pro Mol einer Verbindung der Formel III, worin n gleich Null ist, weniger als 2 Mol 1,2-Dichlor- 1,2-Dibromethan, 1,4-Diclor- oder 1,4-Dibrombuten-(2) verwendet.

Verbindungen der Formel IV, worin p die durchschnittliche Zahl 1 bedeutet und q für Null steht, können beispielsweise hergestellt werden, indem man 2 Mol einer Verbindung der Formel V mit 3 Mol Cyclopentadien in bekannter Weise umsetzt.

Verbindungen der Formel IV, worin p eine durchschnittliche Zahl von 2 bis 100 bedeutet und q für Null steht, können hergestellt werden, indem man (p+1) Mole einer Verbindung der Formel V mit (p+2) Mole Cyclopentadien in bekannter Weise umsetzt.

Verbindungen der Formel IV, worin q eine durchschnittliche Zahl von 1 bis 100 bedeutet, können hergestellt werden, indem man eine Verbindung der Formel VI

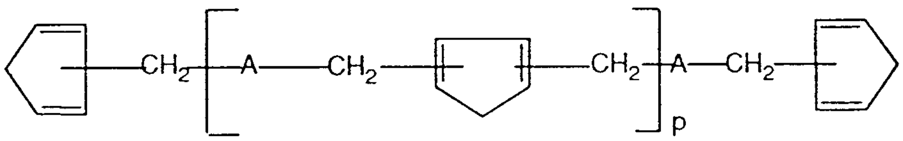 (VI),

worin A und p die gleiche Bedeutung wie in Formel II haben, mit einer Verbindung der Formel III, worin n die Bedeutung von q in Formel II hat, bei einer Temperatur von 50 bis 100°C gemäss einer Diels-Alder-Reaktion umsetzt.

Verbindungen der Formel IV, worin $R_1$ und $R_1$' sowie $R_2$ und $R_2$' nicht nur für Wasserstoffatome sondern zusammen auch für Ethylen stehen, $R_4$, m, p, q und A die gleiche Bedeutung wie in Formel I haben, sind in der Literatur noch nicht beschrieben worden und stellen einen weiteren Erfindungsgegenstand dar.

Die Verbindungen der Formel V sind bekannt und zum Teil im Handel erhältlich, beispielsweise das p-Xylylendichlorid oder das Bis-(chlormethyl)-biphenyl. Aromatische Di-(chlormethyl)-verbindungen können ausserdem durch Chlormethylierung in einfacher Weise hergestellt werden. Die Einführung der Chlormethylgruppe in den aromatischen Kern gelingt in bekannter Weise mit Formaldehyd und Chlorwasserstoff in Gegenwart von Zinkchlorid, Aluminiumchlorid oder Phosphorsäure als Katalysator gemäss der Blancschen Reaktion.

Die Verbindungen der Formel VI stellen ebenfalls bekannte Verbindungen dar und können in bekannter Weise durch Umsetzung von Verbindungen der Formel V mit Cyclopentadien hergestellt werden.

Die Herstellung der erfindungsgemässen Spiro[2,4]hepta-4,6-dien-verbindungen kann, ausgehend vom Bis-(chlormethyl)-benzol (p-Xylylendichlorid) beispielsweise durch folgendes vereinfachtes Reaktionsschema veranschaulicht werden, wobei jedoch in Betracht gezogen werden muss, dass die Umsetzung des Bis-(cyclopentadienyl)-xylols mit 1,2-Dichlorethan nicht nur zum monomeren Bis-(spiro[2,4]hepta-4,6-dienyl)-xylol führt, sondern ein Gemisch erhalten wird, in dem je nach Reaktionsführung im allgemeinen bis zu etwa 10 Gew.-% oligomere Spiroverbindungen enthalten

sein können.

Reaktionsschema:

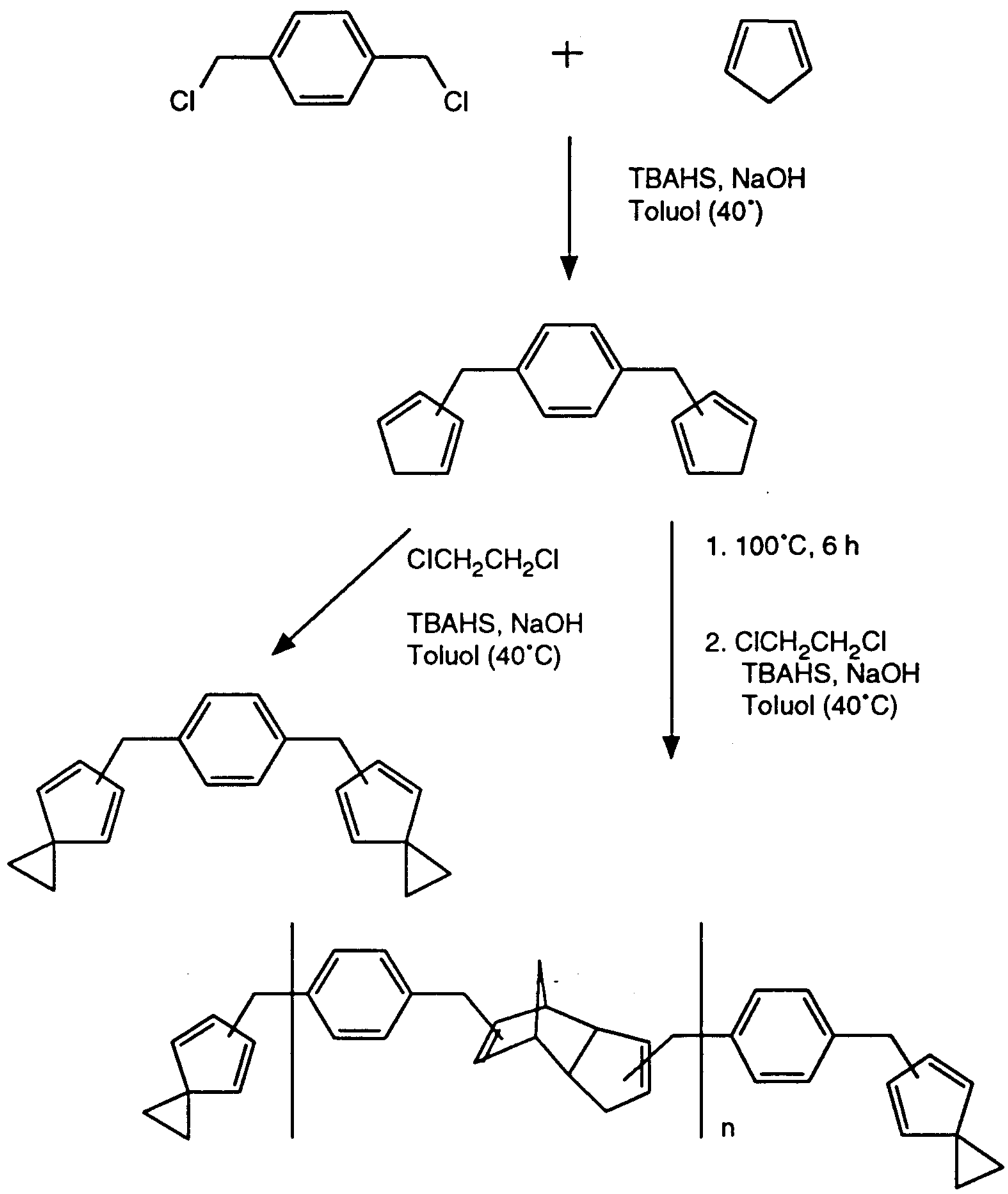


[TBAHS = Tetrabutylammoniumhydrogensulfat]

Durch Reaktion der in Zwischenstufe bei der Umsetzung von beispielsweise Bis-(cyclopentadienyl)-xylol mit 1,2-Dichlorethan entstehenden Mono-(β-chlorethyl)cyclopentadienylverbindung der Formel

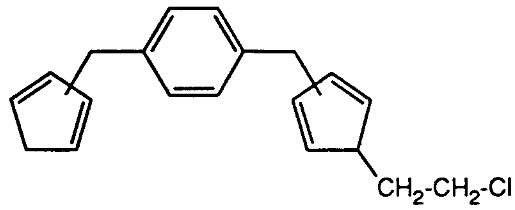

mit einem anderen Bis-(cyclopentadienyl)-xylol können dabei Nebenprodukte der folgenden Formel

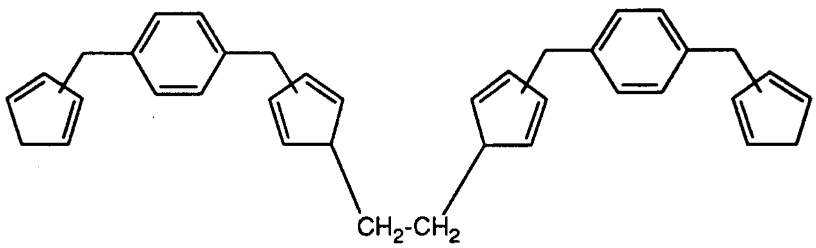

entstehen, die in den erfindungsgemässen Gemischen aber nur in untergeordneten Mengen vorhanden sind. Analoge Nebenverbindungen können auch durch Reaktion der genannten Zwischenverbindung mit Cyclopentadien entstehen.

Bei Verwendung von 1,4-Dichlorbuten-(2) anstelle von 1,2-Dichlorethan werden nach dem oben angegebenen Reaktionsschema unter den gleichen Reaktionsbedingungen ein monomeres und oligomere vinyl-substituierte Spiro[2,4]hepta-4,6-dien-verbindungen der folgenden Formeln erhalten:

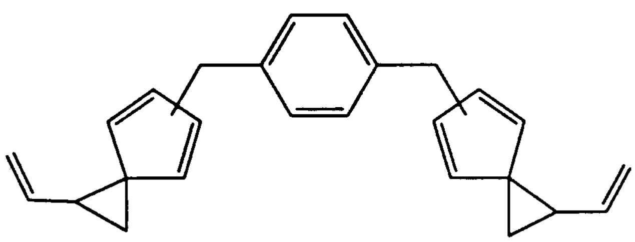

und

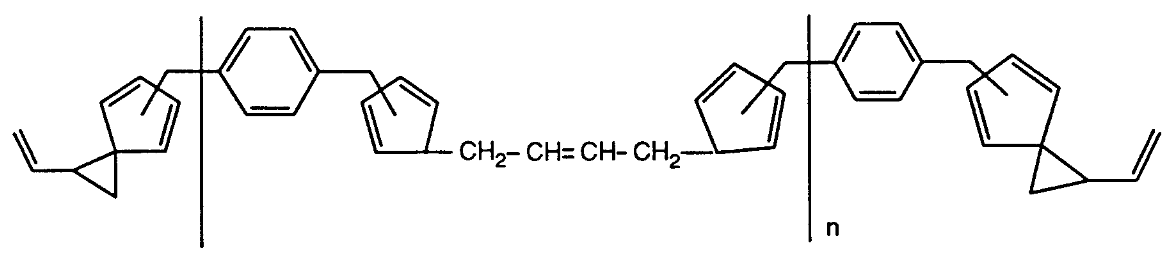

Als Phasentransferkatalysatoren, die bei der Herstellung der erfindungsgemässen Verbindungen eingesetzt werden können, eignen sich beispielsweise quaternäre Ammoniumsalze, wie Tetramethylammoniumchlorid, Tetraethylammoniumchlorid, Benzyltrimethylammoniumchlorid, Benzyltrimethylammoniumacetat, Methyltriethylammoniumchlorid, Tetrabutylammoniumchlorid, Methyltri-$C_8$-$C_{10}$-alkylammoniumchloride (Adogen®464) oder Tetrabutylammoniumsulfat, quaternäre Ammoniumbasen, wie Benzyltrimethylammoniumhydroxid, sowie die Kronenether, wie 12-Kronen-4-ether (1,4,7,10-Tetraaoxacyclododecan), 15-Kronen-5-ether (1,4,7,10,13-Pentaoxacyclopentadecan), 18-Kronen-6-ether, Dibenzo-18-kronen-6-ether, Dibenzo-24-kronen-8-ether, Dibenzo-1,4-dioxa-8, 12-diazacyclopentadecy-5-14-dien, Dicyclohexano-18-kronen-6-ether oder Dicyclohexano-24-kronen-8-ether.

Die quaternären Ammoniumverbindungen und die genannten Kronenether sind bekannte Verbindungen und zum Teil im Handel erhältlich.

Die Herstellung der erfindungsgemässen Verbindungen erfolgt vorzugsweise in Gegenwart von Alkalilösungen, beispielsweise einer wässrigen Kali- oder Natronlaugelösung. In der Regel verwendet man eine 30- bis 80-%ige wässrige Alkalilösung, vorzugsweise eine 40- bis 60-%ige wassrige Natronlauge.

Die erfindungsgemässen Verbindungen können durch Erhitzen auf höhere Temperaturen zu unlöslichen und unschmelzbaren Kunststoffprodukten mit vielseitigen technischen Anwendungsmöglichkeiten ausgehärtet werden. Geeignete Härtungstemperaturen liegen in der Regel bei etwa 130 bis 280°C, vorzugsweise bei 170 bis 250°C, wobei die Aushärtungszeit stark von der Härtungstemperatur abhängt.

Für manche technische Anwendungen ist für die Aushärtung der Zusatz eine Härtungskatalysators vorteilhaft. Geeignet sind z.B. Zugaben einer geringeren Menge eines Peroxids, wodurch man bei 200°C Gelierzeiten von wenigen Minuten erreichen kann. Dabei sind Peroxide, wie Di-tert.-butylperoxid, Di-tert.-butylperoxidbutan, Dilaurylperoxid, Dicumylperoxid und tert.-Butylcumylperoxid in einer Konzentration von 0,01 bis 5 %, vorzugsweise 0,25 bis 0,5 %, bezogen auf das Gewicht der erfindungsgemässen Verbindungen, geeignet.

Es können jedoch auch andere bekannte Härtungsbeschleuniger wie Kobaltnaphthenat, eingesetzt werden. Geeignet sind ferner die Radikale oder Kationen bildende Katalysatoren, wie beispielsweise die Oniumsalze, insbesondere die araliphatischen Sulfoniumsalze, die in der EP-A-0 379 464 beschrieben werden.

Der Ausdruck "Härten", wie er hier gebraucht wird bedeutet die Umsetzung der erfindungsgemässen Verbindungen zu einem vernetzten, unlöslichen und unschmelzbaren Produkt.

Die Herstellung der vernetzten, unschmelzbaren Produkte erfolgt in der Regel unter gleichzeitiger Formgebung, beispielsweise zu Giesskörpern, Laminaten, Pressformstoffen, Verklebungen oder Beschichtungen, wie Oberflächenbeschichtungen.

Die erfindungsgemässen Gemische können im ungefüllten Zustand oder zusammen mit in der Technologie der härtbaren Kunststoffe gebräuchlichen Zusätzen oder Modifizierungsmitteln, wie Füllmitteln, Weichmachern, Lösungsmitteln, Pigmenten, Farbstoffen, Formtrennmitteln, flammhemmenden Stoffen usw., unter Formgebung, nach dem Einbringen in Klebefugen oder als Flächengebilde ausgehärtet werden, wobei gegebenenfalls auch Druck angewendet werden kann. Gegenstand vorliegender Erfindung sind auch die aus den erfindungsgemässen Gemischen durch Härtung hergestellten Formstoffe oder Flächengebilde.

Als flammhemmende Stoffe verwendet man in den erfindungsgemässen Gemischen vorzugsweise phosphorhaltige Verbindungen der Formel VII

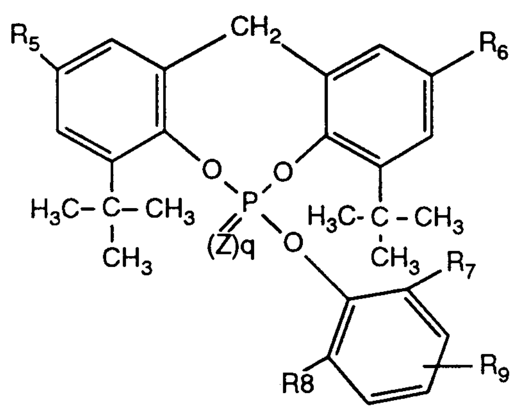

(VII),

worin $R_5$ und $R_6$ unabhängig voneinander je ein Wasserstoffatom, $C_1$-$C_6$-Alkyl, unsubstituiertes oder durch 1 bis 3 $C_1$-$C_4$-Alkylgruppen substituiertes Phenyl oder Naphthyl bedeuten, $R_7$ und $R_8$ unabhängig voneinander je ein Wasserstoffatom oder $C_1$-$C_4$-Alkyl darstellen, wobei $R_7$ und $R_8$ nicht gleichzeitig Wasserstoffatome sind, $R_9$ ein Wasserstoffatom, $C_1$-$C_6$-Alkyl, unsubstituiertes oder durch 1 bis 3 $C_1$-$C_4$-Alkylgruppen substituiertes Phenyl oder Naphthyl, Z ein Sauerstoff- oder Schwefelatom bedeutet und q für null oder die Zahl 1 steht.

Die Verbindungen der Formel VII und deren Verwendung sind aus der EP-A-0 412 936 bekannt und können nach dem dort beschriebenen Verfahren hergestellt werden.

Vorzugsweise finden die erfindungsgemässen Gemische bei der Laminatherstellung Verwendung, da sie einerseits eine sehr gute Haftung auf Metallen, insbesondere Kupfer, zeigen und ausserdem nach den gebräuchlichen Techniken zur Herstellung von beispielsweise kupferkaschierten Platten eingesetzt werden können. Andererseits weisen die aus den erfindungsgemässen Gemische durch Härtung erhaltenen Harze gute dielektrische Eigenschaften auf, welche die

Harze besonders für die Herstellung von kupferkaschierten Schichtstoffen als Basismaterial für gedruckte Schaltungen geeignet machen.

Die erfindungsgemässen Verbindungen können auch zur Herstellung von dünnen Filmen für Schaltungen mit Leiterplatten hoher Dichte, wie z.B. in der Microchips-Industrie (Multichipsmodul-Technologie) verwendet werden.

Der Quotient aus den beiden Molekulargewichtsangaben Mw/Mn kann als Mass für die Grösse der Molekulargewichtsverteilung angesehen werden. Bei einer einheitlichen Verbindung beträgt der Quotient 1 und bei Polymeren ist er stets >1.

Beispiel 1: Spiro[2,4]hepta-4,6-diene auf der Basis von p-Xylylendichlorid.

In einem Sulfierkolben mit mechanischem Rührer, Rückflusskühler, Thermometer und zwei Tropftrichtern werden 87,5 g (0,5 Mol) p-Xylylendichlorid in 750 g Toluol unter $N_2$ gelöst. Dann werden 3,4 g (0,01 Mol) Tetrabutylammoniumhydrogensulfat (TBAHS) zugegeben und die Mischung wird auf 35°C erwärmt. Zu dieser Mischung werden unter kräftigem Rühren 69,4 g (1.05 Mol) frisch destilliertes Cyclopentadien und 320 g einer 50-%igen NaOH-Lösung (4 Mol) gleichzeitig zugetropft, damit die Temperatur bei 30°C bleibt. Die Mischung wird noch 2 Stunden (h) bei 35°C gerührt und 1 h bei 50°C erwärmt. Dann wird sie auf 35°C abgekühlt. Bei dieser Temperatur werden 10,2 g (0,03 Mol) TBAHS zugegeben. Danach werden 98,9 g (1 Mol) 1,2-Dichlorethan und 194 g einer 50-%igen NaOH-Lösung (2,42 Mol) gleichzeitig zugetropft. Die Mischung wird 1 h bei 35°C und 5,5 h bei 50°C gerührt und dann auf Raumtemperatur (RT) abgekühlt. Die wässrige Phase wird abgetrennt. Die organische Phase wird mit 510 g einer 30%-igen $NaHSO_4$-Lösung zu pH = 4 angesäuert und zweimal mit Wasser gewaschen. Die wässrige Phase wird abgetrennt und die organische Phase wird über Natriumsulfat getrocknet und dann filtriert. Das Toluol wird abgedampft.
Man bekommt 99,2 g (70% d.Th.) eines Gemisches von oligomeren Spiro[2,4]hepta-4,6-dienen als braunes Harz.

| Elementaranalyse: | |
|---|---|
| ber. C=92,26% | gef. C=90,82% |
| ber. H = 7,74% | gef. H = 7,53%. |
| Gesamtchlorgehalt: | gef. 0,97% |

$^1$H-NMR-Spektrum:

$$\frac{\text{H cyclopropyl}}{\text{H aromatisch}} = 1,16 \text{ (theor. = 2,0)}$$

Gelpermeationschromatographie: (GPC)
Mn = 456
Mw = 1039.

Beispiel 2: Spiro[2,4]hepta-4,6-diene auf der Basis von 4,4'-Bis-(chlormethyl)-biphenyl.

In einem 12 Liter-Reaktor mit mechanischem Rührer, Rückflusskühler, Thermometer und zwei Tropftrichtem werden 1,256 kg (5,0 Mol) Bis-(chlormethyl)-biphenyl und 34 g (0,1 Mol) TBAHS in 7 kg Toluol bei 35°C unter $N_2$ suspendiert. Zu dieser Suspension werden unter kräftigem Rühren 695 g (10,5 Mol) frisch destilliertes Cyclopentadien und 1,6 kg einer 50%igen NaOH Lösung (20 Mol) gleichzeitig zugetropft, damit die Temperatur zwischen 35 und 38°C bleibt. Die Mischung wird noch 1,5 h bei 35°C gerührt und dann auf 40°C erwärmt. Bei dieser Temperatur werden 102 g (0,3 Mol) TBAHS und 990 g (10 Mol) 1,2-Dichlorethan schnell zugegeben, und dann werden 1,928 kg einer 50%igen NaOH-Lösung (24,1 Mol) in 0,5 h zugetropft. Die Mischung wird 1 h bei 40°C und 2 h bei 50°C gerührt und dann auf Raumtemperatur abgekühlt. Nach der Zugabe von 0,5 kg Wasser wird die wässrige Phase dekantiert und abgetrennt. Die organische Phase wird mit 0,1 kg einer 40%igen $NaHSO_4$-Lösung zu pH = 4 angesäuert. Die wässrige Phase wird abgetrennt und die organische Phase wird über Natriumsulfat getrocknet und dann über Kieselgur filtriert. Das Toluol wird abgedampft.

Man erhält 1,274 kg (78,5 % d. Th.) eines Gemisches von oligomeren Spiro[2,4]hepta-4,6-dienen als braunes Harz.

| Elementaranalyse: | |
|---|---|
| ber. C = 92,77% | gef. C = 92,00% |
| ber. H = 7,23% | gef. H = 7,1 %. |
| Gesamtchlorgehalt: | gef. 0,169%. |
| Hydrolisierbares Chlor: | gef. 0,024%. |

$^1$H-NMR-Spektrum:

$$\frac{\text{H cyclopropyl}}{\text{H aromatisch}} = 0{,}39 \text{ (theor. = 1,0)}$$

GPC: Mn = 544
Mw = 1385.

Beispiel 3:

In einen 2,5 Liter fassenden 5-Halskolben, ausgerüstet mit Schnellrührer, $N_2$-Gaseinlassöffnung, Thermometer, Rückflusskühler und zwei Tropftrichtem, werden 43,75 g (0,25 Mol) p-Xylylendichlorid, 1,7 g (0,005 Mol) TBAHS und 375 g Toluol zugegeben. 34,7 g (0,525 Mol) Cyclopentadien werden in den einen Tropftrichter und 160 g (2,00 Mol) der 50%-igen wässrigen Natronlauge werden in den anderen Tropftrichter eingefüllt. Unter schnellem Rühren und einem kontinuierlichen $N_2$-Gasstrom werden das Cyclopentadien und die Natronlaugelösung gleichzeitig während 1 h in den Kolben getropft, wobei die Temperatur der Reaktionslösung durch äussere Kühlung auf 25°C gehalten wird. Dann wird die Lösung während 2,5 h bei 25°C gerührt und anschliessend während 1,5 h bei 50°C. Danach wird die Lösung auf 25°C abgekühlt.

Das Reaktionsgemisch wird der Diels-Alder-Reaktion unterworfen, indem es während 6 h auf 100°C erhitzt wird. Danach wird die Reaktionslösung auf RT abgekühlt. Zu dieser Lösung werden 5,09 g (0,015 Mol) TBAHS und 97 g einer 50%-igen wässrigen Natronlauge zugegeben. Unter $N_2$-Atmosphäre werden 49,3 g (0,5 Mol) 1,2-Dichlorethan während 30 Minuten kontinuierlich zugetropft, wobei die Reaktionslösung durch äussere Kühlung zwischen 34 bis 40°C gehalten wird. Anschliessend wird die Reaktionslösung noch 6 h bei 50°C gehalten, dann auf Raumtemperatur abgekühlt und mit 800 g $NaHSO_4$ neutralisiert. Die organische Phase wird von der wässrigen Phase abgetrennt, zweimal mit 200 ml Wasser gewaschen und dann über wasserfreiem $Na_2SO_4$ getrocknet. Das Toluol wird aus der getrockneten Toluollösung unter Vakuum bei RT am Rotationsverdampfer entfernt und das zurückbleibende Harz wird für 15 Minuten im Hochvakuum gehalten, damit die restlichen flüchtigen Anteile entfernt werden. Die Ausbeute beträgt 65 % der Theorie an polymeren Spiro[2,4]hepta-4,6-dienen als braunes Harz.

| Elementaranalyse: | |
|---|---|
| ber. C = 92,26 % | gef. C = 89,98 % |
| ber. H = 7,74 % | gef. H = 7,66 %. |
| Gesamtchlorgehalt: | gef. 0,54 %. |

GPC: Mn = 1172
Mw = 7448.

$^1$H-NMR-Spektrum:

$$\frac{\text{H cyclopropyl}}{\text{H aromatisch}} = 0{,}92$$

Viskosität bei 100°C = 44875 mPa·s.

Beispiel 4:

Das im Beispiel 3 erhaltene Harz wird zu Formkörpern durch Aushärtung verarbeitet, indem es in Giessformen während 6 h bei 150°C, 3 h bei 180°C und 3 h bei 220°C erhitzt wird. Die erhaltenen Formkörper weisen folgende Eigenschaften auf:

| | |
|---|---|
| $T_G$ (TMA) | = 289°C |
| Wärmeformbeständigkeit (ISO-R 75) | = 216°C |
| Biegefestigkeit (ISO-R 178) | = 92,6 N/mm$^2$ |
| Bruchdehnung (DIN 53 371) | = 3,0 % |
| Elastizitätsmodul (DIN 53 371) | = 3111 N/mm$^2$ |
| Gewichtsverlust (Temperatur<-> | = 0,5 % bei 310°C |
| gravimetrie =TGA) | = 1,0 % bei 347°C |
| | = 5,0 % bei 417°C |
| Wasseraufnahme | = 0,115 % nach 24 h Wasserlagerung bei 23°C |
| | = 0,134 % nach 30 Minuten Wasserlagerung bei 100°C |
| Dielektrizitätskonstante | = 2,6-2,7 (3 KHz bei 24°C) |
| Dielektrizitätskonstante nach Feuchtigkeitsbehandlung während 800 h bei 85°C und 85 % relativer Luftfeuchtigkeit | = 2,7-2,8 (3 KHz bei 24°C) |
| dielektrischer Verlustfaktor tan δ | = < 0,005 (100 Hz-1 MHz) |

Beispiel 5:

Das gemäss Beispiel 2 erhaltene Harz wird zu Formkörpern durch Aushärtung verarbeitet, indem es in Giessformen während 6 h bei 150°C, 3 h bei 180°C und 3 h bei 220°C erhitzt wird. Die erhaltenen Formkörper weisen folgende Eigenschaften auf:

| | |
|---|---|
| Wasseraufnahme | = 0,246-0,276 % nach 22 h Wasserlagerung bei 25°C |
| | = 0,825-0.839 % nach 1140 h Wasserlagerung bei 25°C |
| Dielektrizitätskonstante Dielektrizitätskonstante nach Feuchtigkeitsbehandlung während 1400 h bei 85°C und 85 % | = 2,83 (3 KHz bei 25°C) |
| relativer Luftfeuchtigkeit | = 2,92-2,96 (3 KHz bei 24°C). |

Beispiel 6: Vinyl-spiro[2,4]heptadiene auf der Basis von p-Xylylendichlorid und 1,4-Dichlorbuten-(2).

In einem Sulfierkolben mit mechanischem Rührer, Rückflusskühler, Thermometer und zwei Tropftrichtern werden 35 g (0,2 Mol) p-Xylylendichlorid in 300 g Toluol unter $N_2$ gelöst Dann werden 1,35 g (0,004 Mol) TBAHS zugegeben und die Mischung wird auf 35°C erwärmt. Zu dieser Mischung werden unter kräftigem Rühren 29,1 g (0,44 Mol) frisch destilliertes Cyclopentadien und 129 g (1,6 Mol) einer 50%-igen NaOH-Lösung gleichzeitig zugestropft, damit die Temperatur zwischen 35 und 40°C bleibt. Die Mischung wird noch 2 h bei 35°C gerührt und über Nacht bei RT gerührt. Die Mischung wird dann filtriert und die organische Phase wird abgetrennt.
In einen wie oben ausgerüsteten Sulfierkolben werden 128 g (1,6 Mol) einer 50%-igen NaOH-Lösung, 6,79 g (0,02 Mol) TBAHS und 160 g Toluol zugegeben und auf 30°C unter $N_2$ erwärmt. Dazu werden unter kräftigem Rühren 50 g (0,4 Mol) 1,4-Dichlorbuten-(2) und die oben in erster Stufe erhaltene organische Phase gleichzeitig zugetropft, damit die Reaktionstemperatur zwischen 30 und 35°C bleibt. Die Mischung wird noch 1 h bei dieser Temperatur gerührt und dann 1 h auf 50°C erwärmt. Sie wird dann auf RT abgekühlt und filtriert, und die wässrige Phase wird abgetrennt. Die organische Phase wird mit einer 30%-igen $NaHSO_4$-Lösung zu pH=4 angesäuert und zweimal mit Wasser gewaschen. Sie wird über Natriumsulfat getrocknet und filtriert, und das Toluol wird dann abgedampft.

Man erhält 53 g (78% der Theorie) eines braunen Harzes.

| Elementaranalyse: | |
|---|---|
| ber. C = 92,26 % | gef. C=90,35 % |
| ber. H = 7,74 % | gef. H = 7,56 %. |
| Gesamtchlorgehalt: | gef. 1,045%. |

$^1$H-NMR-Spektrum:

$$\frac{\text{H cyclopropyl}}{\text{H aromatisch}} = 0{,}6 \text{ (theoretisch} = 1{,}5)$$

GPC: Mn = 706
Mw = 5762.

Beispiel 7

Je 31 g einer Verbindung der Formel VII, worin $R_5$, $R_6$, $R_7$ und $R_8$ je für die Methylgruppe stehen, $R_9$ ein Wasserstoffatom bedeutet und q für null steht (phosphorhaltige Verbindung A) und einer Verbindung der Formel VII, worin $R_5$, $R_6$, $R_7$ und $R_8$ je für die Methylgruppe stehen, $R_9$ ein Wasserstoffatom bedeutet, q für die Zahl 1 steht und Z ein Sauerstoffatom bedeutet (phosphorhaltige Verbindung B), werden zu 500 g einer 62%-igen Lösung der gemäss Beispiel 2 erhaltenen Verbindung in Toluol gegeben. Die Mischungen werden solange rührt, bis die phosphorhaltigen Verbindungen sich vollständig aufgelöst haben. Das Toluol wird dann am Rotationsverdampfer unter Vakuum bei 50°C/30 mbar abdestilliert, und die erhaltene klare Harzlösung wird einem Vakuum von 0.1 mbar während 3 Minuten bei 120°C und 1 Minute bei 150°C ausgesetzt. Die viskose Harzlösung wird in auf 150°C vorgewärmte, 4 mm dicke Aluminiumformen gegossen und während 3 h bei 150°C, 3 h bei 180°C und 3 h bei 200°C ausgehärtet mir einer Nachhärtung von 6 h bei 200°C. Die erhaltenen Proben werden 7 Tage bei 70°C getrocknet, bevor an ihnen der Brennbarkeitstest nach UL[*)]94 vorgenommen wird. Die Proben des mit den phosphorhaltigen Verbindungen A und B flammfest gemachten Harzes gemäss Beisiel 2 ergeben jeweils den Wert V0.

An den Proben, enthaltend die phosphorhaltige Verbindung A, wird ausserden die Dielektrizitätskonstante vor und nach der Wasserlagerung sowie die Wasseraufnahme bestimmt.

| | |
|---|---|
| Dielektrizitätskonstante, trocken Dielektrizitätskonstante, nach 24 h | = 2,9 (3 KHz bei 25°C) |
| Wasserlagerung bei 100°C | = 3,0 (3 KHz bei 25°C) |
| Wasseraufnahme nach 24 h Wasserlagerung bei 100°C | = 0,39%. |

[*)]Underwriter's Laboratories

## Patentansprüche

**Patentansprüche für folgende Vertragsstaaten : CH, DE, FR, GB, IT, LI, NL, SE**

1. Gemische enthaltend im wesentlichen Verbindungen der Formel I oder II

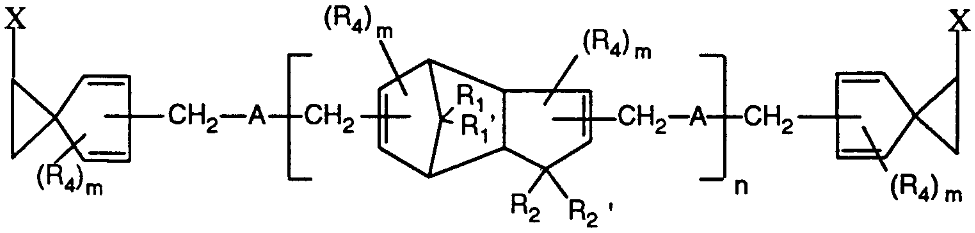

(I) oder

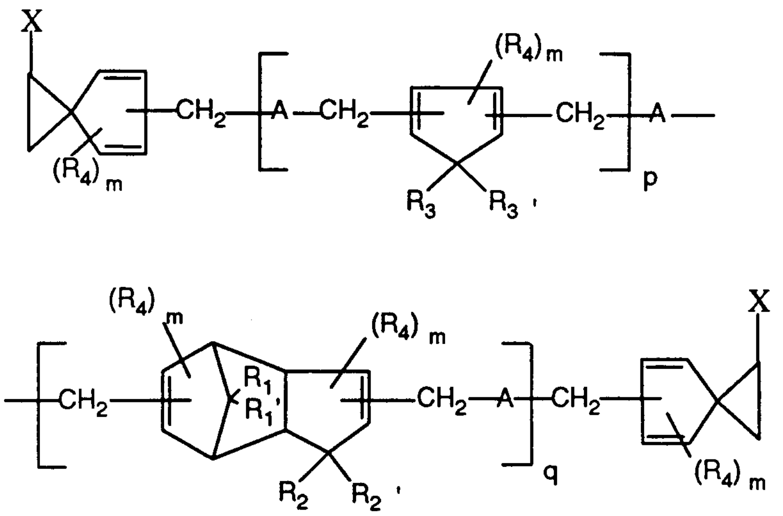

(II),

worin X für ein Wasserstoffatom oder Vinyl steht, A einen unsubstituierten oder durch ein oder mehrere $C_1$-$C_{20}$-Alkyle, Halogenatome oder halogensubstituierte $C_1$-$C_{20}$-Alkyle substituierten zweiwertigen aromatischen Rest bedeutet, $R_1$, $R_1$', $R_2$, $R_2$', $R_3$ und $R_3$' je für ein Wasserstoffatom oder $R_1$ und $R_1$', $R_2$ und $R_2$' und $R_3$ und $R_3$' zusammen je für Ethylen, $R_4$ je für ein $C_1$-$C_6$-Alkyl, Phenylalkyl mit 1-6 C-Atomen im Alkylrest oder Trialkylsilyl mit je 1-6 C-Atomen im Alkylrest, m für Null oder die Zahl 1 oder 2, n für Null oder eine durchschnittliche Zahl von 1 bis 200, p für eine durchschnittliche Zahl von 1 bis 100 und q für Null oder eine durchschnittliche Zahl von 1 bis 100 stehen.

2. Gemische enthaltend im wesentlichen Verbindungen der Formel I oder II gemäss Anspruch 1, worin X für ein Wasserstoffatom steht, und A, $R_1$, $R_1$', $R_2$, $R_2$', $R_3$, $R_3$', $R_4$ m und n die gleiche Bedeutung wie im Anspruch 1 haben.

3. Gemische enthaltend im wesentlichen Verbindungen der Formel I gemäss Anspruch 1, worin A, $R_1$, $R_1'$, $R_2$, $R_2'$, $R_3$, $R_3'$, $R_4$ m und n die gleiche Bedeutung wie im Anspruch 1 haben.

4. Gemische gemäss Anspruch 1, worin A für einen unsubstituierten oder einen $C_1$-$C_4$-alkylsubstituierten Rest der Formel

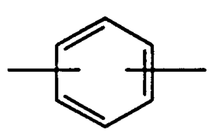 , 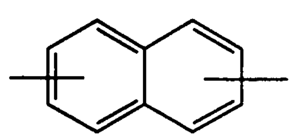 oder 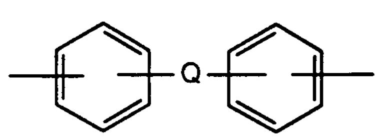

steht, worin Q die direkte Bindung, $-(CH_2)_r-$, $-C(CH_3)_2-$, -O-, -CO-, -S- oder $-SO_2-$ bedeutet, wobei r für eine Zahl von 1 bis 12 steht, $R_1$, $R_1'$, $R_2$ und $R_2'$ die gleiche Bedeutung wie im Anspruch 1 haben, m für Null oder die Zahl 1

oder 2 steht, wobei $R_4$ je ein Methyl oder Ethyl bedeutet, und n für Null oder eine durchschnittliche Zahl von 1 bis 20 steht.

**5.** Gemische aus Verbindungen der Formel I gemäss Anspruch 1, worin A für Phenylen oder Biphenylen steht, $R_1$, $R_1'$, $R_2$ und $R_2'$ die gleiche Bedeutung wie in Anspruch 1 haben und n für Null oder eine durchschnittliche Zahl von 1 bis 20 steht.

**6.** Gemische gemäss Anspruch 5, worin n für Null oder eine durchschnittliche Zahl von 1 bis 10 steht.

**7.** Verfahren zur Herstellung von Gemischen gemäss Anspruch 1, dadurch gekennzeichnet, dass man Verbindungen der Formel III oder IV

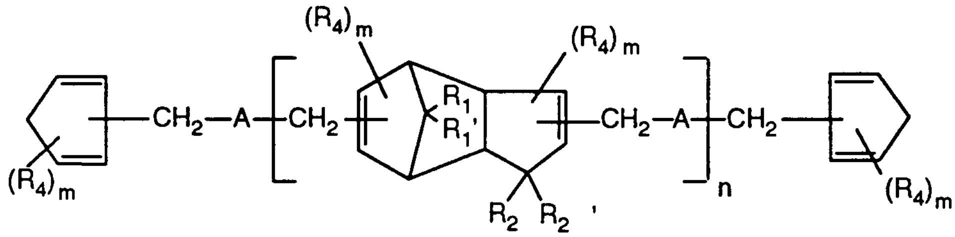

(III) oder

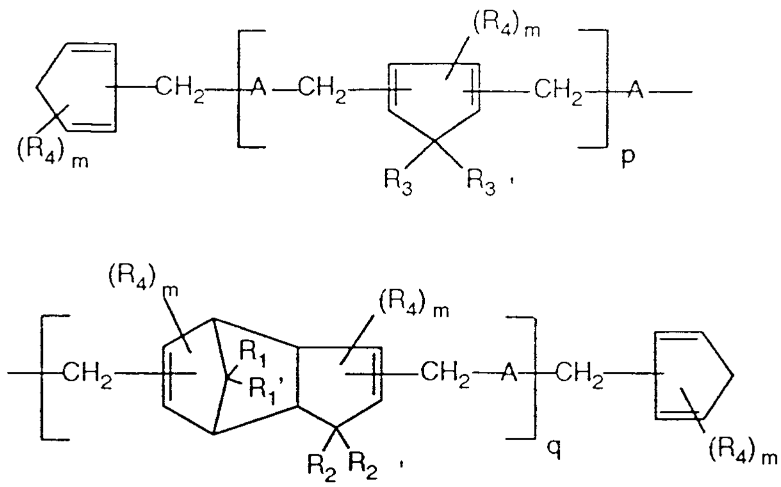

(IV),

worin A, $R_1$, $R_1'$, $R_2$, $R_2'$, $R_3$, $R_3'$, $R_4$, m, n, p und q die gleiche Bedeutung wie in Formel I und II gemäss Anspruch 1 haben, mit 1,2-Dichlor-, 1,2-Dibromethan, 1,4-Dichlor- oder 1,4-Dibrombuten-(2) in Gegenwart eines Phasentransferkatalysators und von Alkali im Temperaturbereich von 20 bis 50°C umsetzt, wobei man pro 1 Mol der Verbindung der Formel III oder IV mindestens 0,1 Mole 1,2-Dichlor-, 1,2-Dibromethan, 1,2-Dichlor- oder 1,2 Dibrombuten-(2) einsetzt.

**8.** Verfahren gemäss Anspruch 7, dadurch gekennzeichnet, dass man mindestens 2 Mole 1,2-Dichlor-, 1,2-Dibromethan, 1,4-Dichlor- oder 1,4-Dibrombuten-(2) pro 1 Mol der Verbindung der Formel III oder IV einsetzt.

**9.** Die aus den Gemischen gemäss Anspruch 1 durch Härtung erhaltenen Formstoffe oder Flächengebilde.

**10.** Laminate, hergestellt unter Verwendung der Gemische gemäss Anspruch 1.

**Patentansprüche für folgenden Vertragsstaat : ES**

1. Verfahren zur Herstellung von Gemischen enthaltend im wesentlichen Verbindungen der Formel I oder II

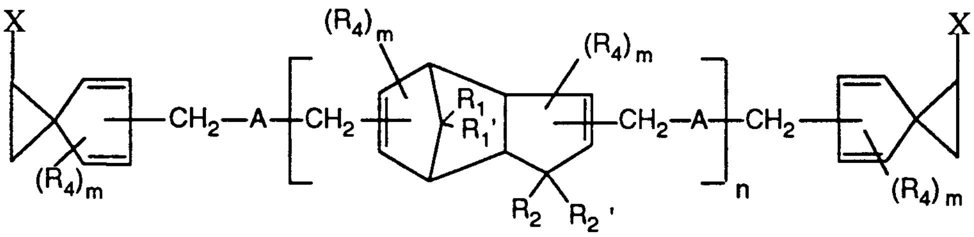

(I) oder

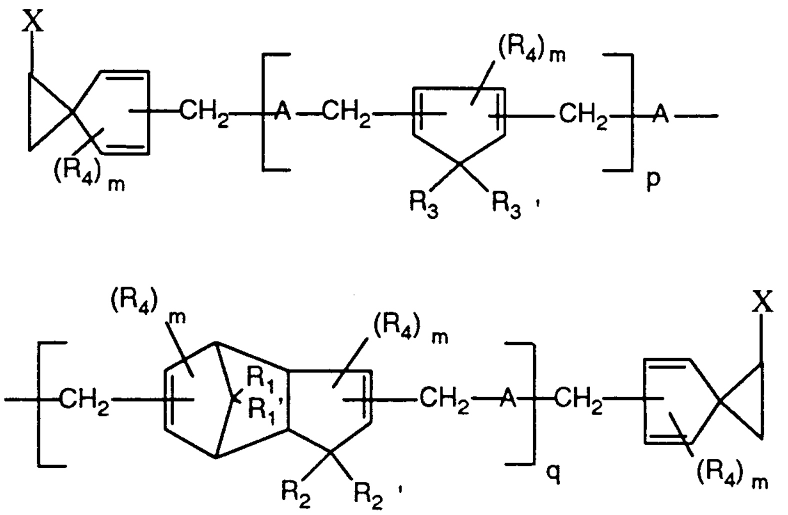

(II),

worin X für ein Wasserstoffatom oder Vinyl steht, A einen unsubstituierten oder durch ein oder mehrere $C_1$-$C_{20}$-Alkyle, Halogenatome oder halogensubstituierte $C_1$-$C_{20}$-Alkyle substituierten zweiwertigen aromatischen Rest bedeutet, $R_1$, $R_1'$, $R_2$, $R_2'$, $R_3$ und $R_3'$ je für ein Wasserstoffatom oder $R_1$ und $R_1'$, $R_2$ und $R_2'$ und $R_3$ und $R_3'$ zusammen je für Ethylen, $R_4$ je für ein $C_1$-$C_6$-Alkyl, Phenylalkyl mit 1-6 C-Atomen im Alkylrest oder Trialkylsilyl mit je 1-6 C-Atomen im Alkylrest, m für Null oder die Zahl 1 oder 2, n für Null oder eine durchschnittliche Zahl von 1 bis 200, p für eine durchschnittliche Zahl von 1 bis 100 und q für Null oder eine durchschnittliche Zahl von 1 bis 100

stehen, dadurch gekennzeichnet, dass man Verbindungen der Formel III oder IV

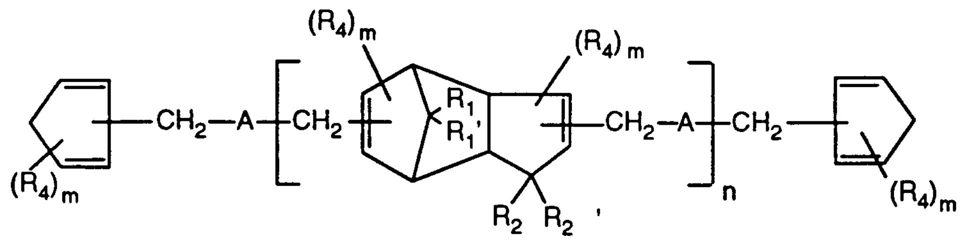

(III) oder

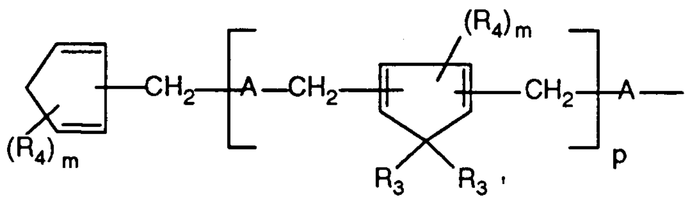

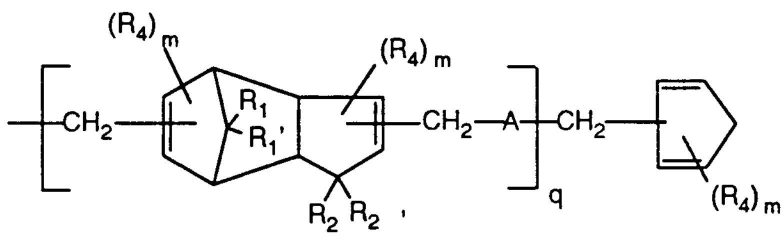

(IV),

worin A, $R_1$, $R_1'$, $R_2$, $R_2'$, $R_3$, $R_3'$, $R_4$, m, n, p und q die gleiche Bedeutung wie in Formel I und II haben, mit 1,2-Dichlor-, 1,2-Dibromethan, 1,4-Dichlor- oder 1,4-Dibrombuten-(2) in Gegenwart eines Phasentransferkatalysators und von Alkali im Temperaturbereich von 20 bis 50°C umsetzt, wobei man pro 1 Mol der Verbindung der Formel III oder IV mindestens 0,1 Mole 1,2-Dichlor-, 1,2-Dibromethan, 1,2-Dichlor- oder 1,2 Dibrombuten-(2) einsetzt.

**2.** Verfahren gemäss Anspruch 1, dadurch gekennzeichnet, dass man Verbindungen der Formel III oder IV mit 1,2-Dichlor- oder 1,2-Dibromethan umsetzt.

**3.** Verfahren gemäss Anspruch 1, dadurch gekennzeichnet, dass man mindestens 2 Mole 1,2-Dichlor-, 1,2-Dibromethan, 1,4-Dichlor- oder 1,4-Dibrombuten-(2) pro 1 Mol der Verbindung der Formel III oder IV einsetzt.

## Claims

**Claims for the following Contracting States : CH, DE, FR, GB, IT, LI, NL, SE**

1. A mixture essentially comprising compounds of formula I or II

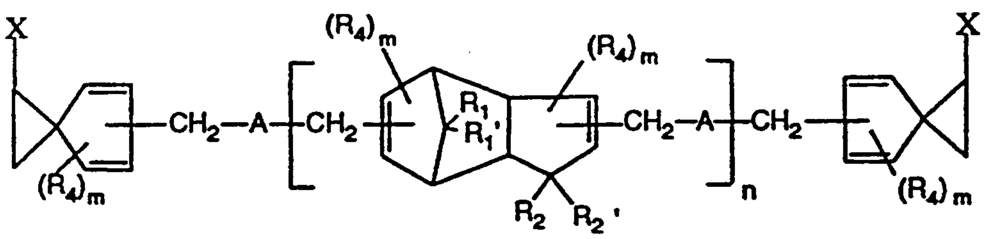

(I) or

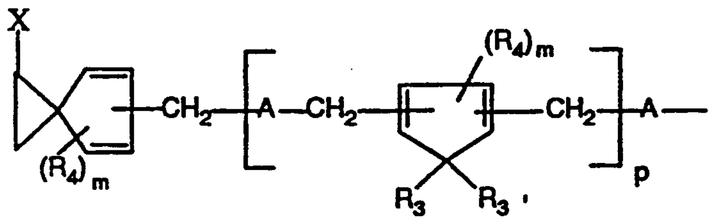

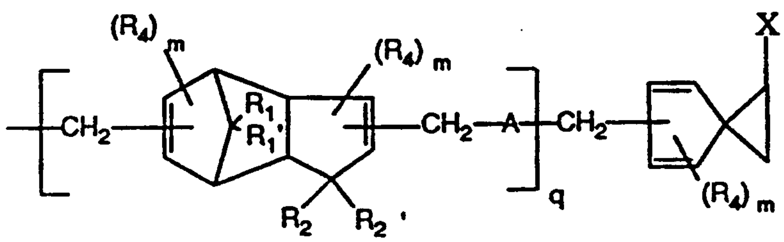

(II),

wherein X is a hydrogen atom or vinyl, A is a divalent aromatic radical which is unsubstituted or substituted by one or more $C_1$-$C_{20}$alkyls, halogen atoms or halogen-substituted $C_1$-$C_{20}$alkyls, $R_1$, $R_1'$, $R_2$, $R_2'$, $R_3$ and $R_3'$ are each a hydrogen atom, or $R_1$ and $R_1'$, $R_2$ and $R_2'$ and $R_3$ and $R_3'$ are each together ethylene, $R_4$ is in each case $C_1$-$C_6$alkyl, phenylalkyl containing 1 to 6 carbon atoms in the alkyl moiety or is trialkylsilyl containing 1 to 6 carbon atoms in each alkyl moiety, m is zero, 1 or 2, n is zero or an average number from 1 to 200, p is an average number from 1 to 100 and q is zero or an average number from 1 to 100.

2. A mixture essentially comprising compounds of formula I or II according to claim 1, wherein X is a hydrogen atom, and A, $R_1$, $R_1'$, $R_2$, $R_2'$, $R_3$, $R_3'$, $R_4$, m and n are as defined in claim 1.

3. A mixture essentially comprising compounds of formula I according to claim 1, wherein A, $R_1$, $R_1'$, $R_2$, $R_2'$, $R_3$, $R_3'$, $R_4$, m and n are as defined in claim 1.

4. A mixture according to claim 1, wherein A is an unsubstituted or a $C_1$-$C_4$alkyl-substituted radical of formula

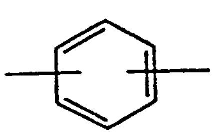

,

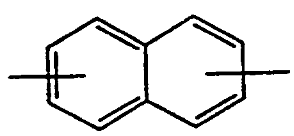

or

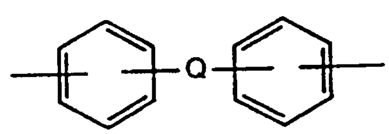

wherein Q is a direct bond, $-(CH_2)_r$, $-C(CH_3)_2-$, -O-, -CO-, -S- or $-SO_2-$, where r is a number from 1 to 12, $R_1$, $R_1'$, $R_2$ and $R_2'$ are as defined in claim 1, m is zero, 1 or 2, and $R_4$ is in each case methyl or ethyl, and n is zero or an average number from 1 to 20.

5. A mixture comprising compounds of formula I according to claim 1, wherein A is phenylene or biphenylene, $R_1$, $R_1'$, $R_2$ and $R_2'$ are as defined in claim 1, and n is zero or an average number from 1 to 20.

6. A mixture according to claim 5, wherein n is zero or an average number from 1 to 10.

7. A process for the preparation of a mixture according to claim 1, which comprises reacting compounds of formula III or IV

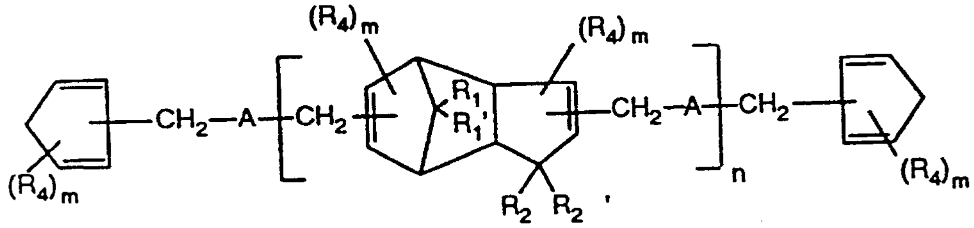

(III) or

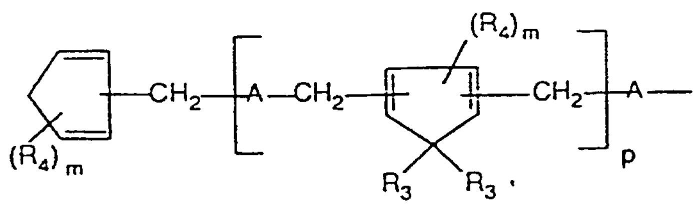

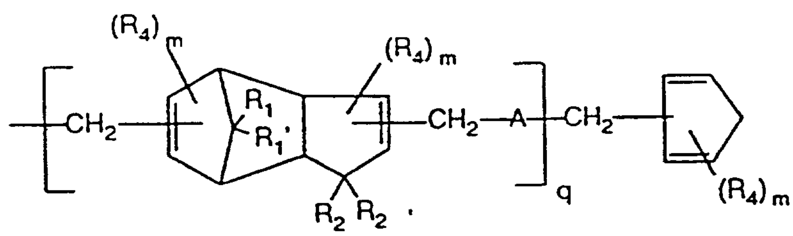

(IV),

wherein A, $R_1$, $R_1'$, $R_2$, $R_2'$, $R_3$, $R_3'$, $R_4$, m, n, p and q are as defined for formulae I and II according to claim 1, with 1,2-dichloroethane, 1,2-dibromoethane, 1,4-dichloro- or 1,4-dibromobut-2-ene in the presence of a phase transfer catalyst and of alkali and in the temperature range from 20 to 50°C, using at least 0.1 mol of 1,2-dichloroethane, 1,2-dibromoethane, 1,4-dichloro- or 1,4-dibromobut-2-ene per 1 mol of compound of formula III or IV.

8. A process according to claim 7, which comprises using at least 2 mol of 1,2-dichloroethane, 1,2-dibromoethane, 1,4-dichloro- or 1,4-dibromobut-2-ene per 1 mol of compound of formula III or IV.

9. A moulded article or flat structure obtained from a mixture according to claim 1 by curing.

10. A laminate prepared using a mixture according to claim 1.

**Claims for the following Contracting State : ES**

1. A process for producing a mixture essentially comprising compounds of formula I or II

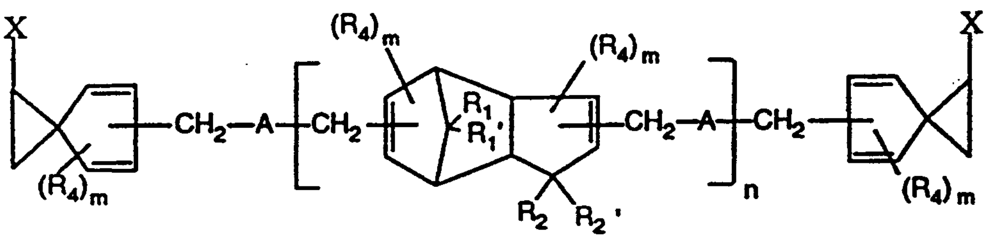

(I) or

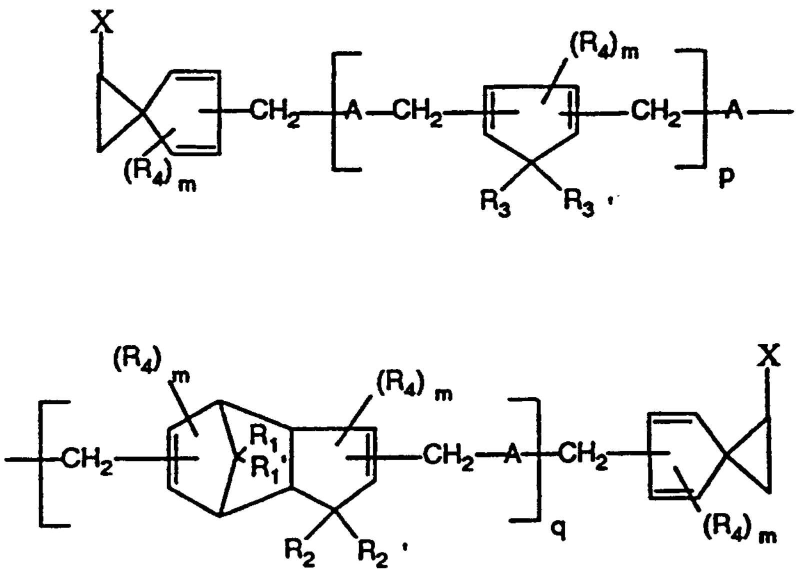

(II),

wherein X is a hydrogen atom or vinyl, A is a divalent aromatic radical which is unsubstituted or substituted by one or more $C_1$-$C_{20}$alkyls, halogen atoms or halogen-substituted $C_1$-$C_{20}$alkyls, $R_1$, $R_1$', $R_2$, $R_2$', $R_3$ and $R_3$' are each a hydrogen atom, or $R_1$ and $R_1$', $R_2$ and $R_2$' and $R_3$ and $R_3$' are each together ethylene, $R_4$ is in each case $C_1$-$C_6$alkyl, phenylalkyl containing 1 to 6 carbon atoms in the alkyl moiety or is trialkylsilyl containing 1 to 6 carbon atoms in each alkyl moiety, m is zero, 1 or 2, n is zero or an average number from 1 to 200, p is an average number from 1

to 100 and q is zero or an average number from 1 to 100, which comprises reacting compounds of formula III or IV

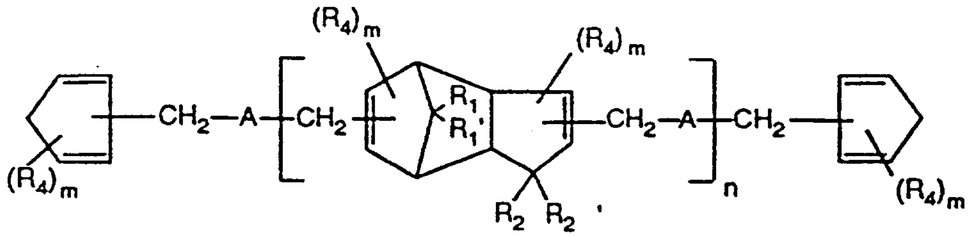

(III) or

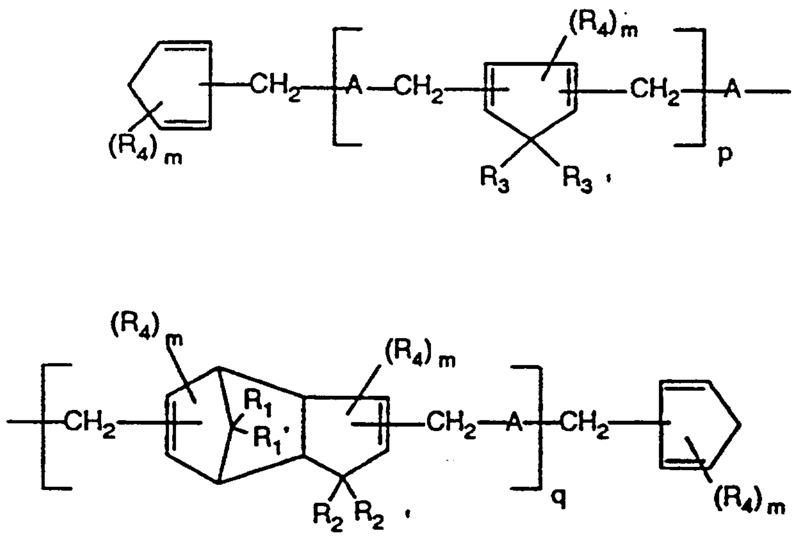

(IV),

wherein A, $R_1$, $R_1$', $R_2$, $R_2$', $R_3$, $R_3$', $R_4$, m, n, p and q are as defined for formulae I and II, with 1,2-dichloroethane, 1,2-dibromoethane, 1,4-dichloro- or 1,4-dibromobut-2-ene in the presence of a phase transfer catalyst and of alkali and in the temperature range from 20 to 50°C, using at least 0.1 mol of 1,2-dichloroethane, 1,2-dibromoethane, 1,4-dichloro- or 1,4-dibromobut-2-ene per 1 mol of compound of formula III or IV.

**2.** A process according to claim 1 wherein compounds of formula III or IV are reacted with 1,2-dichloroethane or 1,2-dibromoethane.

**3.** A process according to claim 1, which comprises using at least 2 mol of 1,2-dichloroethane, 1,2-dibromoethane, 1,4-dichloro- or 1,4-dibromobut-2-ene per 1 mol of compound of formula III or IV.

## Revendications

**Revendications pour les Etats contractants suivants : CH, DE, FR, GB, IT, LI, NL, SE**

**1.** Mélanges contenant essentiellement des composés de formule I ou II :

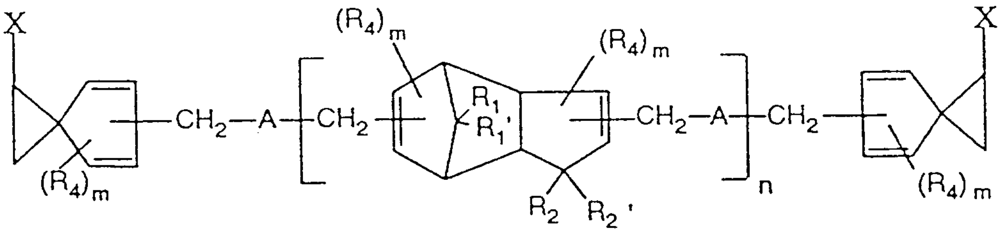 (I)

ou

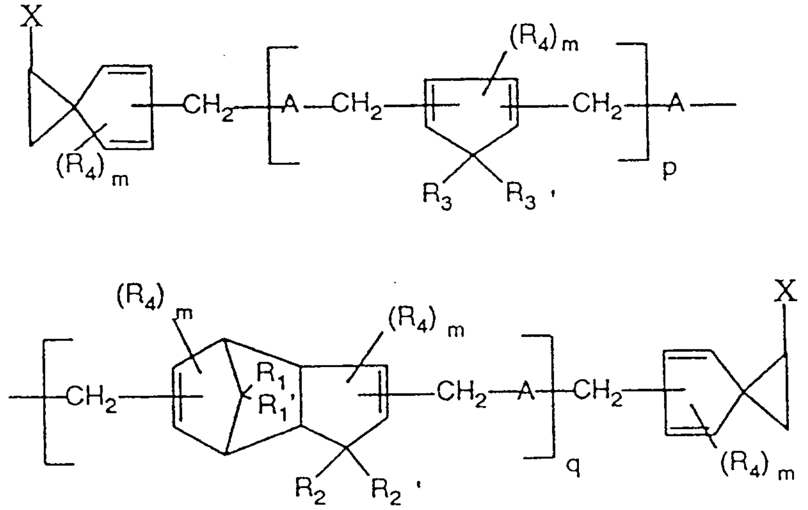 (II),

où X est un atome d'hydrogène ou le radical vinyle, A est un radical aromatique divalent, non substitué, ou une ou deux fois substitué par des substituants alkyle en $C_1$-$C_{20}$, des atomes d'halogène ou des substituants alkyle en $C_1$-$C_{20}$ halogénés, $R_1$, $R_1'$, $R_2$, $R_2'$, $R_3$ et $R_3'$ sont chacun un atome d'hydrogène, ou bien $R_1$ et $R_1'$, $R_2$ et $R_2'$ et $R_3$ et $R_3'$, forment ensemble des groupes éthylène, chaque radical $R_4$ est un groupement alkyle en $C_1$-$C_6$, phénylalkyle ayant de 1 à 6 atomes de carbone dans le fragment alkyle ou trialkylsilyle ayant chacun de 1 à 6 atomes de carbone dans le fragment alkyle, m vaut 0 ou représente le nombre 1 ou 2, n vaut 0 ou représente en moyenne un nombre de 1 à 200, p est un nombre valant en moyenne de 1 à 100, et q vaut 0 ou représente en moyenne un nombre de 1 à 100.

**2.** Mélanges contenant essentiellement des composés de formule I ou II selon la revendication 1, dans lesquelles X est un atome d'hydrogène, et A, $R_1$, $R_1'$, $R_2$, $R_2'$, $R_3$, $R_3'$, $R_4$, m et n ont les mêmes significations que dans la revendication 1.

**3.** Mélanges contenant essentiellement des composés de formule I selon la revendication 1, dans laquelle A, $R_1$, $R_1'$, $R_2$, $R_2'$, $R_3$, $R_3'$, $R_4$, m et n ont les mêmes significations que dans la revendication 1.

**4.** Mélanges selon la revendication 1, dans lesquels A est un radical non substitué, ou substitué par des substituants alkyle en $C_1$-$C_4$, de formule :

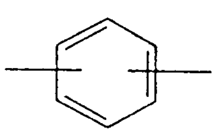 , 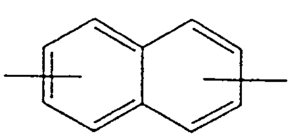 ou 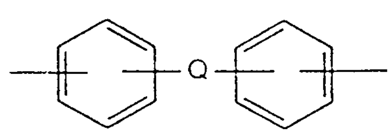

où Q est une liaison directe, $-(-CH_2-)_r-$, $-C(CH_3)_2-$, -O-, -CO-, -S- ou $-SO_2-$, où r est un nombre de 1 à 12, $R_1$, $R_1'$, $R_2$ et $R_2'$ ont les mêmes significations que dans la revendication 1, m vaut 0 ou représente le nombre 1 ou 2, chaque radical $R_4$ étant un radical méthyle ou éthyle, et n vaut 0 ou est en moyenne un nombre de 1 à 20.

**5.** Mélanges de composés de formule I selon la revendication 1, dans laquelle A est le phénylène ou le biphénylène, $R_1$, $R_1'$, $R_2$ et $R_2'$ ont les mêmes significations que dans la revendication 1, et n vaut 0 ou est en moyenne un nombre de 1 à 20.

**6.** Mélanges selon la revendication 5, dans lesquels n vaut 0 ou est en moyenne un nombre de 1 à 10.

**7.** Procédé de préparation de mélanges selon la revendication 1, caractérisé en ce qu'on fait réagir des composés de formule III ou IV :

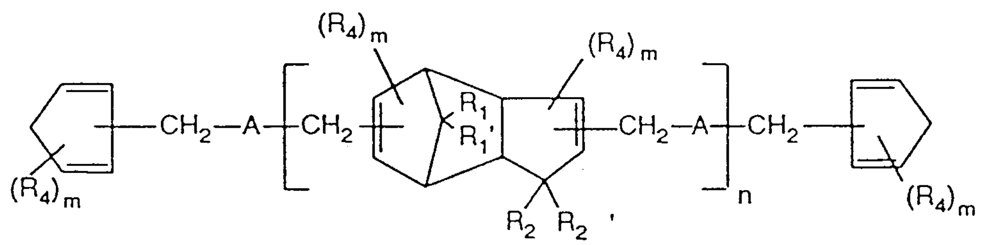

(III) ou

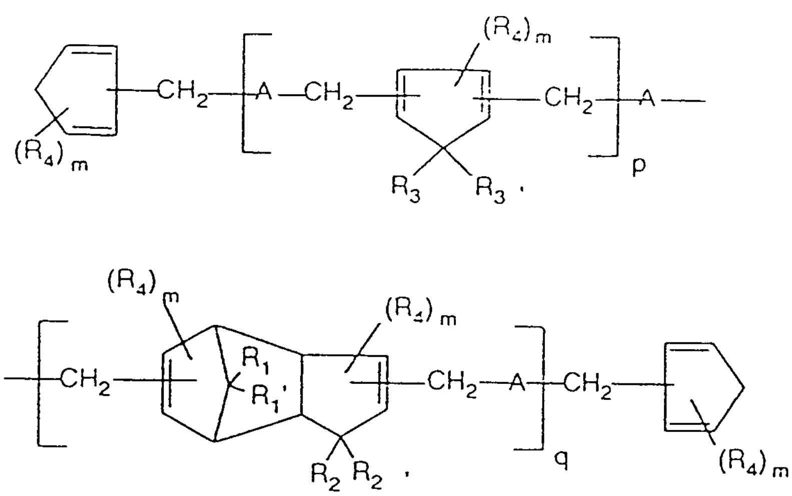

(IV),

dans lesquelles A, $R_1$, $R_1'$, $R_2$, $R_2'$, $R_3$, $R_3'$, $R_4$, m, n, p et q ont les mêmes significations que dans les formules I et II selon la revendication 1, avec du 1,2-dichloro- ou du 1,2-dibromométhane ou du 1,4-dichloro- ou du 1,4-dibromobutène-(2), en présence d'un catalyseur de transfert de phase et d'un alcali, dans un intervalle de températures de 20 à 50°C, auquel cas on utilise par mole du composé de formule III ou IV au moins 0,1 mole de 1,2-dichloro- ou de 1,2-dibromométhane ou de 1,2-dichloro- ou du 1,2-dibromobutène-(2).

**8.** Procédé selon la revendication 7, caractérisé en ce qu'on utilise par mole du composé de formule III ou IV au moins 2 moles de 1,2-dichloro- ou de 1,2-dibromométhane ou de 1,4-dichloro- ou de 1,4-dibromobutène-(2).

**9.** Objets moulés ou structures bidimensionnelles obtenus par durcissement à partir des mélanges selon la revendication 1.

**10.** Stratifiés fabriqués par utilisation des mélanges selon la revendication 1.

**Revendications pour l'Etat contractant suivant : ES**

**1.** Procédé pour préparer des mélanges contenant essentiellement des composés de formule I ou II :

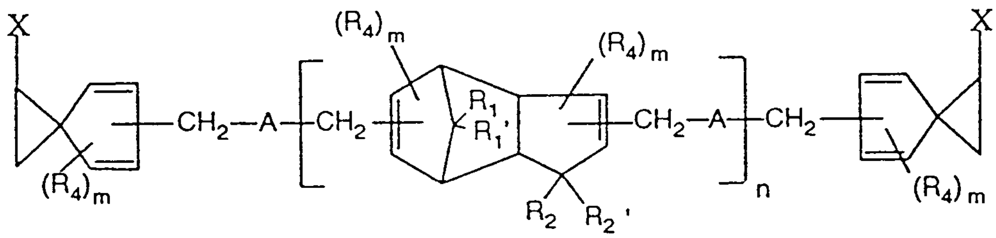

(I)

ou

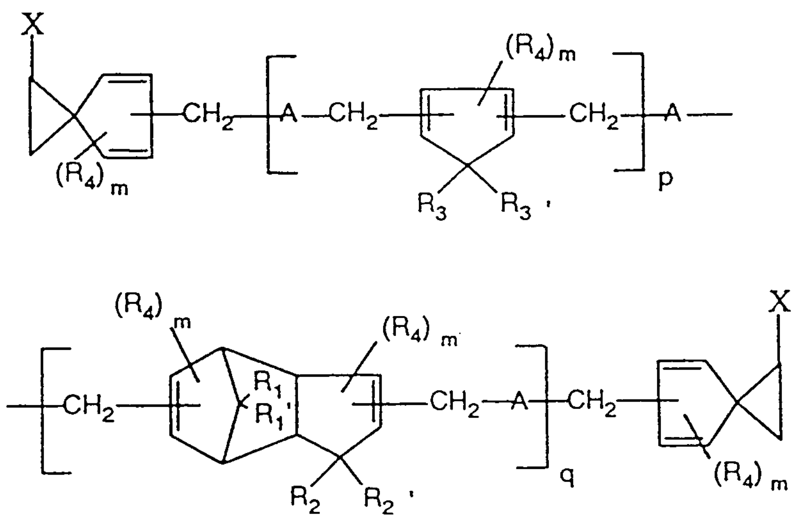

(II),

où X est un atome d'hydrogène ou le radical vinyle, A est un radical aromatique divalent, non substitué, ou une ou deux fois substitué par des substituants alkyle en $C_1$-$C_{20}$, des atomes d'halogène ou des substituants alkyle en $C_1$-$C_{20}$ halogénés, $R_1$, $R_1'$, $R_2$, $R_2'$, $R_3$ et $R_3'$ sont chacun un atome d'hydrogène, ou bien $R_1$ et $R_1'$, $R_2$ et $R_2'$ et $R_3$ et $R_3'$, forment ensemble des groupes éthylène, chaque radical $R_4$ est un groupement alkyle en $C_1$-$C_6$, phénylalkyle ayant de 1 à 6 atomes de carbone dans le fragment alkyle ou trialkylsilyle ayant chacun de 1 à 6 atomes de carbone dans le fragment alkyle, m vaut 0 ou représente le nombre 1 ou 2, n vaut 0 ou représente en moyenne un nombre de 1 à 200, p est un nombre valant en moyenne de 1 à 100, et q vaut 0 ou représente en moyenne un nombre de

1 à 100, caractérisé en ce qu'on fait réagir des composés de formule III ou IV :

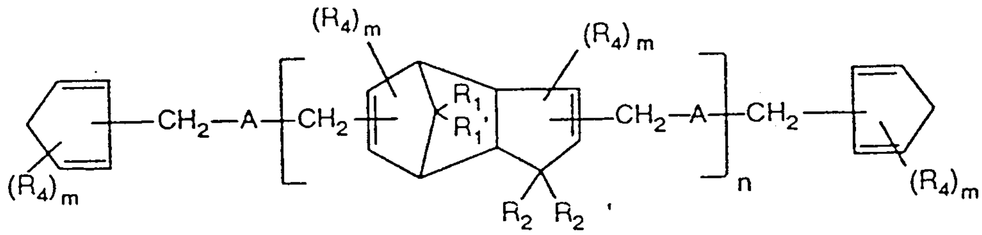

(III) ou

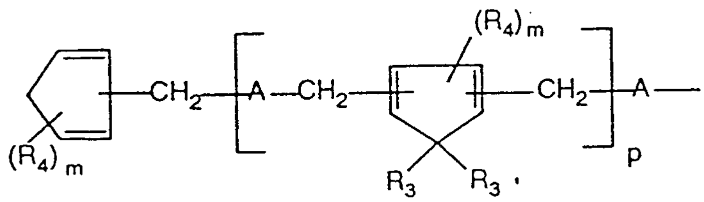

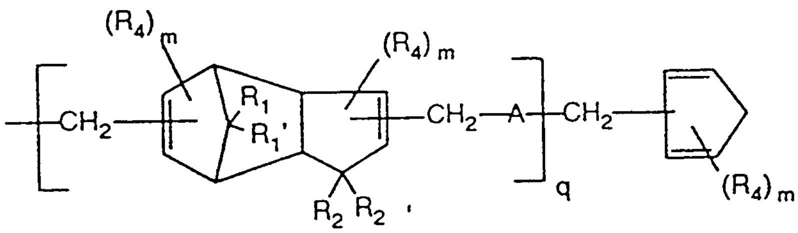

(IV),

dans lesquelles A, $R_1$, $R_1'$, $R_2$, $R_2'$, $R_3$, $R_3'$, $R_4$, m, n, p et q ont les mêmes significations que dans les formules I et II, avec du 1,2-dichloro- ou du 1,2-dibromométhane ou du 1,4-dichloro- ou du 1,4-dibromobutène-(2), en présence d'un catalyseur de transfert de phase et d'un alcali, dans un intervalle de températures de 20 à 50°C, auquel cas on utilise par mole du composé de formule III ou IV au moins 0,1 mole de 1,2-dichloro- ou de 1,2-dibromométhane ou de 1,2-dichloro- ou du 1,2-dibromobutène-(2).

**2.** Procédé selon la revendication 1, caractérisé en ce qu'on fait réagir des composés de formule III ou IV avec du 1,2-dichloro- ou du 1,2-dibromométhane.

**3.** Procédé selon la revendication 1, caractérisé en ce qu'on utilise par mole du composé de formule III ou IV au moins 2 moles de 1,2-dichloro- ou de 1,2-dibromométhane ou de 1,4-dichloro- ou de 1,4-dibromobutène-(2).